# EUROPEAN PATENT APPLICATION

(11) **EP 4 386 772 A1**
(43) Date of publication of application: **19.06.2024**
(21) Application number: 22213765.5
(22) Date of filing: 15.12.2022
(51) Int. Cl.: G16H 20/40, G16H 30/40, G16H 40/63, G16H 10/60, G16H 50/50

(54) **NEUROMONITORING NAVIGATION**

(71) Applicant: Koninklijke Philips N.V., 5656 AG Eindhoven (NL)
(72) Inventor: GRASS, Michael, Eindhoven (NL); SCHRAUWEN, Jelle, Eindhoven (NL); HAASE, Hans Christian, 5656AG Eindhoven (NL); HOLTHUIZEN, Ronaldus Frederik Johannes, Eindhoven (NL)
(74) Representative: Philips Intellectual Property & Standards

(57) **Abstract**

The present invention relates to neuromonitoring navigation in interventional procedures. For an improved navigation, a device (10) for neuromonitoring navigation in interventional procedures is provided that comprises a data input (12), a data processor (14) and an output interface (16). The data input is configured to receive a sequence of neuromonitoring signals (18) related to an interventional medical procedure in a region of interest of a subject, the procedure involving at least one interventional device; and to receive image data representing an anatomy (20) of the region of interest of the subject during the interventional medical procedure; and to provide a 3D model (22), the 3D model provided as at least one of the group comprising a 3D anatomical model and a 3D device shape model. The data processor is configured to register a 3D model to the image data forming an augmented image of the subject; and to assign the signals of the sequence of neuromonitoring signals to procedural steps of the interventional medical procedure; and to allocate the procedural steps to the anatomy of the augmented image; and to compute a visual indicator of the neuromonitoring signals, the visual indicator reflecting the procedural steps, and to integrate the visual indicator within the anatomy in the augmented image thereby for generating neuromonitoring navigation (26). The output interface is configured to output the neuromonitoring navigation.

## Description

### FIELD OF THE INVENTION

The present invention relates to interventional procedures and relates in particular to a device for neuromonitoring navigation in interventional procedures, to a system for neuromonitoring navigation in interventional procedures and to a method for neuromonitoring navigation in interventional procedures.

### BACKGROUND OF THE INVENTION

Neuromonitoring may be provided, as an example, during minimal invasive procedures. During pedicle screw placement, for supporting precise placement of the screws, or other devices, imaging based navigation is provided for the user. Neural monitoring is used to identify possible damages to the spinal cord. However, it has been shown that during complex interventional procedures with a plurality of varying parameters, the outcome signal of the neural monitoring is cumbersome to relate to the procedure itself.

### SUMMARY OF THE INVENTION

There may thus be a need for improved navigation.

The object of the present invention is solved by the subject-matter of the independent claims; further embodiments are incorporated in the dependent claims. It should be noted that the following described aspects of the invention apply also for the device for neuromonitoring navigation in interventional procedures, for the system for neuromonitoring navigation in interventional procedures and for the method for neuromonitoring navigation in interventional procedures.

According to the present invention, a device for neuromonitoring navigation in interventional procedures is provided. The device comprises a data input, a data processor and an output interface. The data input is configured to receive a sequence of neuromonitoring signals related to an interventional medical procedure in a region of interest of a subject, the procedure involving at least one interventional device. The data input is also configured to receive image data representing an anatomy of the region of interest of the subject during the interventional medical procedure. The data input is further configured to provide a 3D model, the 3D model provided as at least one of the group comprising a 3D anatomical model and a 3D device shape model. The data processor is configured to register a 3D model to the image data forming an augmented image of the subject. The data processor is also configured to assign the signals of the sequence of neuromonitoring signals to one or more procedural steps of the interventional medical procedure, wherein each procedural step is associated with at least a part of the anatomy. The data processor is also configured to compute a visual indicator of the neuromonitoring signals, and to integrate the visual indicator with the associated part of the anatomy in the augmented image thereby generating neuromonitoring navigation information.

In certain examples, an output interface is configured to output the neuromonitoring navigation information including the augmented image.

As an effect, an information link is provided between the procedure and its trackable influence on the neural structure of the subject.

As an advantage, for example during pedicle screw placement, the enhanced image data thus provides support in avoiding any damage of the spinal cord. The enhanced image data is applied to guarantee its integrity and support neuromonitoring navigation.

According to an example, the data processor is configured to plot the neuromonitoring signals along a procedural axis. The visual indicator reflects a correlation of the temporal and the spatial changes.

This further facilitates the user's understanding of the correlation.
According to an example, for the assigning, the data input is configured to provide procedural data that comprises information about procedural steps of the interventional medical procedure.

According to an example, the data processor is configured to detect at least one operation axis of the interventional device based on current images. The data processor is configured to compute the visual indicator as representing the neuromonitoring signals over a period of operation along the operation axis.

According to an example, for the assigning, the data input is configured, for the image data, to provide image data as intraprocedural imaging acquired during the procedure. The data processor is configured to identify procedure steps and/or device movements in a field of view based on the intraprocedural imaging. The data processor is also configured to relate changes in neurophysiological measurements to the procedure steps and/or device movements.

According to an example, the data processor is configured to register at least one model of a vertebra to the imaging information used to guide a device insertion during the procedure.

This allows to provide an improved visual anatomical context for the respective neuromonitoring navigation.

According to an example, the data processor is configured to register at least one model of a pedicle screw to the imaging information used to guide a device insertion during the procedure.

This allows to provide an improved visual device context for the respective neuromonitoring navigation.

According to an example, the data processor is configured to detect changes in the neuromonitoring signal exceeding a predetermined threshold and to adapt the visual indicator according to the detected changes. The output interface is configured to output adapted neuromonitoring navigation information.

According to the present invention, also a system for neuromonitoring navigation in interventional procedures is provided. The system comprise a device for neuromonitoring navigation in interventional procedures according to one of the preceding examples, an imaging device, a neuromonitoring device and a display device. The imaging device is configured to provide the image data representing the anatomy of the region of interest of the subject during the interventional medical procedure. The neuromonitoring device is configured to provide the sequence of neuromonitoring signals related to an interventional medical procedure in the region of interest of the subject, the procedure involving at least one interventional device. The display device is configured to present the neuromonitoring navigation information.

According to an example, to provide the image data, the imaging device is an X-ray imaging device. Alternatively or in addition, the imaging device is an optical camera device.

According to the present invention, also a method for neuromonitoring navigation in interventional procedures is provided. The method comprises the following steps:
- receiving a sequence of neuromonitoring signals related to an interventional medical procedure in a region of interest of a subject, the procedure involving at least one interventional device.
- receiving image data representing an anatomy of the region of interest of the subject during the interventional medical procedure.
- registering a 3D model to the image data forming an augmented image of the subject, the 3D model provided as at least one of the group comprising a 3D anatomical model and a 3D device shape model.
- assigning the signals of the sequence of neuromonitoring signals to one or more procedural steps of the interventional medical procedure.
- allocating the procedural steps to corresponding parts of the anatomy.
- computing a visual indicator of the assigned neuromonitoring signals and integrating the visual indicator with the associated anatomy in the augmented image thereby generating neuromonitoring navigation information.
- According to an aspect, current image data and model image data are combined with neuro-signals which are related to the procedural steps. For neuromonitoring navigation, an image is presented that comprises a time axis with respect to the procedural steps.

According to an aspect, the above neuromonitoring navigation is applicable to prosthesis placement procedures like pedicle screw placements,

According to a further aspect, the above neuromonitoring navigation is applicable to ENT (ear, nose and throat) surgery procedure and/or AAA (abdominal aortic aneurysm repair) procedure.

According to another aspect, the above neuromonitoring navigation is applicable to coronary interventions and structural heart interventions.

As an example for the coronary procedures, treatment of micro-thrombi is provided. Regarding the micro-thrombi, examples of the resulting strategy could be either that an operator can consider to administer lysis or similar pharmacological treatment once micro-thrombi are detected, or that an operator can consider to abort further treatment to limit further risks to the patient.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for neuromonitoring navigation in interventional procedures.
Fig. 2 shows an example of a system for neuromonitoring navigation in interventional procedures.
Fig. 3 shows basic steps of an example of a method for neuromonitoring navigation in interventional procedures.
Fig. 4 shows another example of a display showing neuromonitoring navigation information with a visual indicator of the neuromonitoring signals.
Fig. 5, Fig. 6 and Fig. 7 show further examples of a display showing neuromonitoring navigation information with a visual indicator of the neuromonitoring signals.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

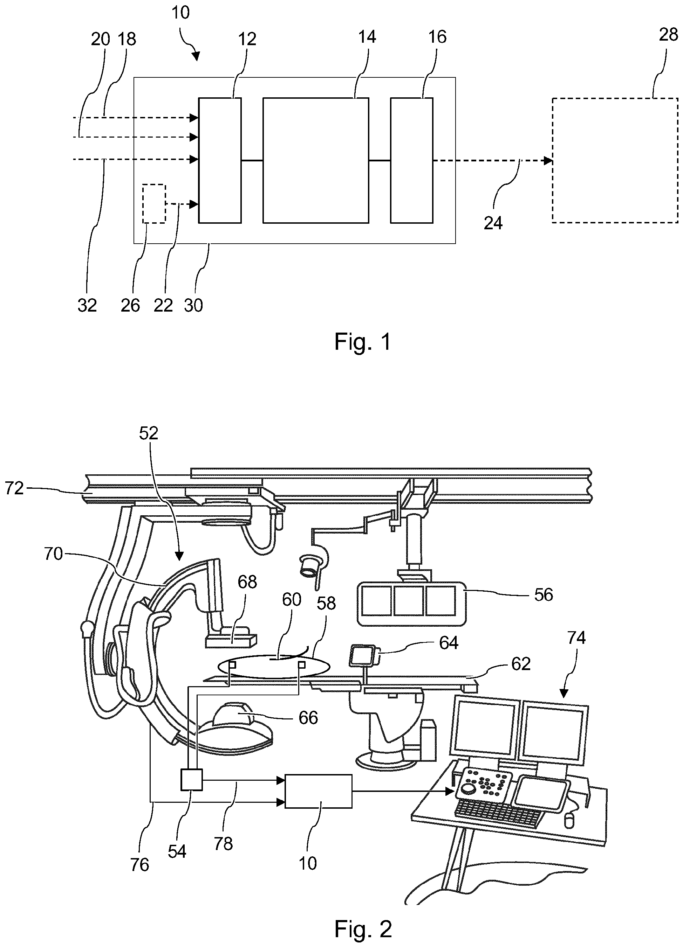

Fig. 1 schematically shows an example of a device 10 for neuromonitoring navigation in interventional procedures. The device 10 comprises a data input 12, a data processor 14 and an output interface 16. The data input 12 is configured to receive a sequence of neuromonitoring signals 18 related to an interventional medical procedure in a region of interest of a subject, the procedure involving at least one interventional device. The data input 12 is also configured to receive image data 20 representing an anatomy of the region of interest of the subject during the interventional medical procedure. The data input 12 is further configured to provide a 3D model 22, the 3D model 22 provided as at least one of the group comprising a 3D anatomical model and a 3D device shape model. The data processor 14 is configured to register a 3D model to the image data forming an augmented image of the subject. The data processor 14 is configured to assign the signals of the sequence of neuromonitoring signals to one or more procedural steps of the interventional medical procedure. Each procedural step is associated with at least a part of the anatomy of the augmented image. The data processor 14 is further configured to compute a visual indicator of the neuromonitoring signals, and to integrate the visual indicator with the associated anatomy in the augmented image thereby generating neuromonitoring navigation information 24. The output interface 16 is configured to output the neuromonitoring navigation information 24.

The neuromonitoring signals 18 are indicated with a first arrow in broken lines; the image data 20 is indicated with a second arrow in broken lines; the 3D model 22 is indicated with a third arrow in broken lines; and the neuromonitoring navigation information 24 is indicated with a fourth arrow in broken lines.

As an option, the 3D model 22 is received from a data storage 26, indicated with a first frame in broken lines. Alternatively or in addition, the 3D model 22 is generated from 3D image data as part of the image data 20 received.

As a further option, in addition or alternatively, the neuromonitoring navigation information 24 is shown on a display unit 28, indicated with a second frame in broken lines.

The data input 12, the data processor 14 and the output interface 16 can be arranged in a common or integrated manner, as indicated with a frame 30, or can be arranged separately.

The term "data input" relates to providing or supplying data for data processing steps. The data input 12 can also be referred to as image data input. The data input 12 can also be referred to as data supply, as image data supply, as image input, as input unit or simply as input. In an example, the data input 12 is data-connectable to an imaging source arrangement. In an example, the data input 12 is data-connected to the data processor 14.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input 12 and the output interface 16.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the controller can be provided by the output interface 16.

In an option, the device for neuromonitoring navigation in interventional procedures also comprises the display unit 28 to show the neuromonitoring navigation information 24 to a user.

The term "neuromonitoring signals" relates to neurophysiological information that is acquired during the interventional medical procedure. The neuromonitoring can also be referred to as neurophysiological sensing and monitoring.

The term "interventional medical procedure" relates to a procedure in which a device is inserted in the region of interest, such as the insertion of pedicle screws.

The term "neuromonitoring navigation" relates to providing navigation support that also provides neurophysiological information in form of monitoring these at the same time and generating a correlation between the steps of procedure, e.g. current action, and the detected neurophysiological signal. Accordingly, "neuromonitoring navigation information" relates to any information that is able to convey such navigation support to a user, such as a physician performing the interventional procedure.

The term "image data representing an anatomy of the region of interest" relates to medical image data of the anatomy.

The term "3D model" relates to 3D image data of a model of the device and/or the anatomy.

The term "augmented image" relates to e.g. live image data of the subject, such as X-ray or optical image data being acquired during an interventional procedure, enhanced or enriched with additional information in form of the 3D model. The augmented image thus provides more than just the current/live image(s).

The term "to assign" relates to mapping the signals of the sequence to the procedural steps. For example, when a plurality of screws are inserted, respective temporal parts of the signals of the sequence are assigned to corresponding screws when the respective screw is being inserted.

The term "visual indicator" relates to visual information to be presented to a user, for example by means of an overlay to the augmented image.

The term "to integrate" relates to combining the visual indicator with the augmented image data. The visual indicator is placed such that a spatial context or correlation with the associated part of the anatomy is provided.

The term "to detect" relates to identifying the signals that are different to others for at least a predetermined amount, for example having a deviation from an expected value that is above a threshold.

The term "to adapt" relates to changing the visual appearance of the visual indicator.

The visual indicator can also be referred to as visual allocator.

The visual indicator is reflecting at least a part of the interventional medical procedure.

The term "signals related to" refers to signals measured during the procedure, i.e. live or current signals.

In an example, the sequence of neuromonitoring signals are live signals.

In an example, 3D shape models are used to integrate the neuromonitoring signals during the procedure. 3D shape models, such as anatomical models or device models, are registered to 2D and 3D imaging information. In an option, this is used to guide minimally invasive procedures.

In an option, the 3D shape models, in combination with live image, are used to integrate, display, and analyze neurophysiological information associated to procedure steps that are performed and devices that are used throughout the intervention.

The resulting setup can be used to optimize a minimally invasive procedure, e.g. including the training of a system for intraprocedural risk detection and risk prediction.

In an option, the data processor 14 is configured to plot the neuromonitoring signals along a procedural axis. The visual indicator reflects a correlation of the temporal and the spatial changes.

An example for the procedural axis is the insertion depth of a pedicle screw. The neuromonitoring signals are plotted along, i.e. against or over the depth. In an example, the visual indicator is integrated next to (the illustration based on) the model of the screw.

In an example, the visual indicator reflects a correlation of the temporal changes of the neuromonitoring signals over time of the procedure and the spatial changes due to a change of the location of the device, i.e. a change of the spatial relation of the anatomy and the device.

In an option of the example of Fig. 1, for the assigning, the data input 12 is configured to provide procedural data 32 that comprises information about procedural steps of the interventional medical procedure.

In an option, the data processor 14 is configured to detect at least one operation axis of the interventional device based on current images. The data processor 14 is also configured to compute the visual indicator as representing the neuromonitoring signals over a period of operation along the operation axis.

In an example, the intervention and a radial deployment direction is a first operation axis. As a second operation axis, a length of an inflatable balloon for deploying stents is provided. In an option, a balloon for dilation is provided, wherein the operating parameter of the balloon provide a third operation axis.

In an example, the procedural data comprises visual or acoustic information identifying different steps of the interventional medical procedure.

In an option, for the assigning, the data input 12 is configured, for the image data, to provide image data as intraprocedural imaging acquired during the procedure. The data processor 14 is configured to identify procedure steps and/or device movements in a field of view based on the intraprocedural imaging, and to relate changes in neurophysiological measurements to the procedure steps and/or device movements.

An example are C-arm based minimally invasive procedures.

Besides fluoroscopy X-ray imaging, also CT imaging, ultrasound imaging and MR imaging are provided.

In an option, the data processor 14 is configured to detect changes in the neuromonitoring signal exceeding a predetermined threshold and to adapt the visual indicator according to the detected changes. The output interface 16 is configured to output an adapted neuromonitoring navigation.

In an example, it is provided the step of detecting changes in the neuromonitoring signal exceeding a predetermined threshold and further the step of adapting the visual indicator according to the detected changes.

Fig. 2 shows an example of a system 50 for neuromonitoring navigation in interventional procedures. The system 50 comprises an example of the device 10 for neuromonitoring navigation in interventional procedures according to one of the preceding examples. The system 50 further comprises an imaging device 52, a neuromonitoring device 54 and a display device 56. The imaging device 52 is configured to provide the image data representing the anatomy of the region of interest of a subject 58 during the interventional medical procedure. The neuromonitoring device 54 is configured to provide the sequence of neuromonitoring signals related to an interventional medical procedure in the region of interest of the subject 58, the procedure involving at least one interventional device 60. The display device 56 is configured to present the neuromonitoring navigation information.

As an example, the display device 56 is provided as a monitor arrangement.

The subject 58 is arranged on a subject support 62. A graphic user interface 64 is indicated as an option.

As an example, the imaging device 52 is provided as an X-ray imaging device with an X-ray source 66 and an X-ray detector 68 mounted to opposite ends of a C-arm 70 that is movably mounted to a ceiling support 72.

A console 74 is shown in the right foreground, the console 74 being provided for operation of the equipment. The console 74 is equipped with various interface devices like keyboard, mouse, trackpad and control knobs, as well as display units.

A first communication line 76 indicates a data flow from the imaging device 52 to the device 10 for neuromonitoring navigation. A second communication line 76 indicates a data flow from the neuromonitoring device 54 to the device 10 for neuromonitoring navigation. The communication can be wire-bound or wireless.

The neuromonitoring device 54 is shown in a simplified manner with a first and second electrode to measure the neural signals.

In an option of the system, initial imaging is performed prior to the procedure to identify vertebral levels, the initial imaging comprising at least one of the group of 2D, multiple 2D or 3D cone beam X-ray imaging, MR imaging and ultrasound imaging.

Fig. 2 shows as an option of the system that, to provide the image data, the imaging device 52 is an X-ray imaging device. In another option, not shown in details, to provide the image data, the imaging device 52 is an optical camera device.

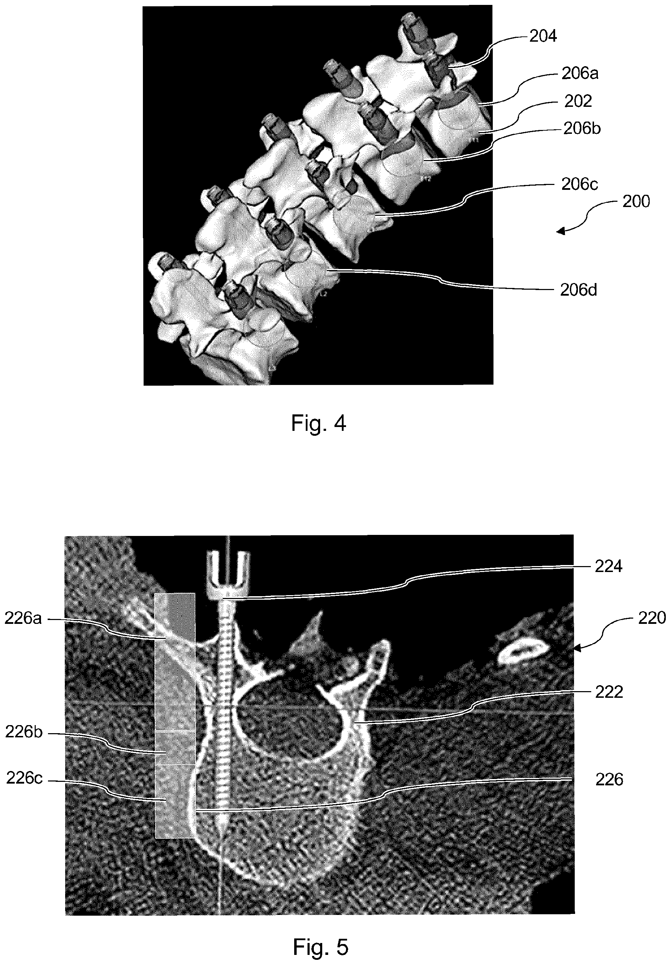

In an option, the procedure is a minimally invasive placement of a plurality of pedicle screws (see also Fig. 4 and Fig. 5). The data processor 14 is configured to identify at least two separate procedural steps of inserting at least one pedicle screw into an associated vertebra or associated vertebrae of the spine, and to assign separate neuromonitoring signals to each of the at least two separate steps.

In an example, the screws of the plurality of screws are interrelated to each other, e.g. forming a common support concept or a common interventional therapeutic measure. In one example, a common data stream is provided reflecting the effects of all of the screws. In another example, several data streams are provided reflecting the different effects of one or some of the screws. In an example, a data stream is provided for each screw.

When inserting the screws, a detection of which screw is operated is provided. This allows the spatial assignment of a data stream for the period of the respective operation, e.g. movement. By plotting the spatial assignment over time, also the depth of the screw can be determined and the neuromonitoring signals can be plotted over the depth.

In an example, the visual indicator is provided having a longitudinal shape assigned to the operation of the device, e.g. the changes of the signal over the screw depth.

In an option, the procedure is provided as a minimally invasive pedicle screw placement in a C-arm based hybrid operation room setting.

In another option, the approach is applied for other minimally invasive procedures that have a risk of compromising neurophysiological signal integrity.

In an example, it is provided the steps of:
- setting up neurophysiological measurements prior to the intervention;
- documenting a place and type of measurement in a user interface;
- sending a temporal data stream to the C-arm system.

In another option, the procedure is an ENT (ear, nose and throat) surgery procedure.

In another option, the procedure is an AAA (abdominal aortic aneurysm repair) procedure.

In a further option, the procedure is a coronary intervention. As an example, neuro signals are monitored during coronary stent placement, for instance detecting early neurological changes prior to a dissection caused by over-inflating a calcified lesion. Or in case of effects of micro-embolization, neuromonitoring ensures that no subsequent intervention needs to be done, as treatment of possible emboly can be done during the initial procedure once they are noticed.

A further example is the case of micro emboli that cause a signal change.

As an option, a delay between the monitoring signal to procedure step correlation is provided, for example depending if a change is expected to occur instantly or with a short delay, like in a traveling clot.

In an option, the data processor 14 is configured to register at least one model of a vertebra to the imaging information used to guide a device insertion during the procedure.

In another option, the data processor 14 is configured to register at least one model of a pedicle screw to the imaging information used to guide a device insertion during the procedure.

In an example, the data input 12 is configured to provide the image data as:
i) X-ray images; and/or
ii) optical camera images.

In an example, the X-ray images are provided as 2D X-ray images, such as fluoroscopy images. In another example, the X-ray images are provided as 3D X-ray images, such as CT image data.

In an example, the optical camera images, i.e. optical camera data, is provided as 2D optical images.
In an option, the data processor 14 is configured to provide spatial tracking for at least one source by the neuromonitoring signals.

For example, if a sensor, e.g. a patch, is moved, a change in the neuromonitoring signals occurs.

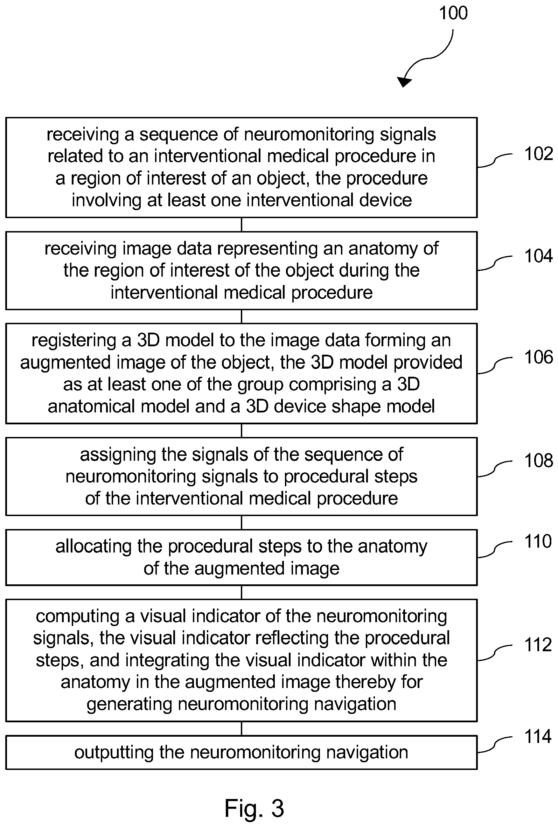

Fig. 3 shows basic steps of an example of a method 100 for neuromonitoring navigation in interventional procedures. The method 100 comprises the following steps: In a first step 102, a sequence of neuromonitoring signals is received related to an interventional medical procedure in a region of interest of a subject, the procedure involving at least one interventional device. In a second step 104, image data is received representing an anatomy of the region of interest of the subject during the interventional medical procedure. In a third step 106, a 3D model is registered to the image data forming an augmented image of the subject. The 3D model is provided as at least one of the group comprising a 3D anatomical model and a 3D device shape model. In a fourth step 108, the signals of the sequence of neuromonitoring signals are assigned to one or more procedural steps of the interventional medical procedure. In a fifth step 110, the procedural steps are associated with at least a part of the anatomy. In a sixth step 112, a visual indicator of the neuromonitoring signals is computed and further integrated with the associated anatomy in the augmented image thereby generating neuromonitoring navigation information. Further, in a seventh step 114, it is provided to output the neuromonitoring navigation information, e.g. by displaying the augmented image on a monitor.

In an example of the method 100, the neuromonitoring signals are plotted along a procedural axis. Further, the visual indicator reflects a correlation of the temporal and the spatial changes.

In another example of the method 100, for the assigning, procedural data is provided that comprises information about procedural steps of the interventional medical procedure.

In a further example of the method 100, at least one operation axis of the interventional device is detected based on current images. Further, the visual indicator is representing the neuromonitoring signals over a period of operation along the operation axis.

In an example of the method 100, for the assigning it is provided:
- for the image data, providing image data as intraprocedural imaging acquired during the procedure;
- identifying procedure steps and/or device movements in a field of view based on the intraprocedural imaging; and
- relating changes in neurophysiological measurements to the procedure steps and/or device movements.

In a still further example of the method 100, the procedure is a minimally invasive placement of a plurality of pedicle screws. Further, at least two separate steps of inserting at least one pedicle screw are identified and neuromonitoring signals are assigned to the two separate steps.

In an example of the method 100, initial imaging is performed prior to the procedure to identify vertebral levels, the initial imaging comprising at least one of the group of 2D, multiple 2D or 3D cone beam X-ray imaging.

In another example of the method 100, at least one model of a vertebrae is registered to the imaging information and used to guide a device insertion during the procedure.

In a still further example of the method 100, the image data is provided as X-ray images and/or optical camera images.

In an example of the method 100, spatial tracking is provided for at least one source by the neuromonitoring signals.

In an example of the method 100, it is provided the steps of:
- detecting changes in the neuromonitoring signal exceeding a predetermined threshold and adapting the visual indicator according to the detected changes; and
- outputting an adapted neuromonitoring navigation.

Once the intervention starts and devices (k-wires, pedicle screws, etc.) are applied and inserted, the neurophysiological information is monitored, and changes are documented on top of the anatomical and/or device shape model to document at which procedure step changes in the signal occur. Since the intervention is an image guided procedure, steps and live location data can be derived from the imaging information. From the imaging information (X-ray and/or optical camera) the live location data for anatomy and devices is extracted through 2D or 3D feature point tracking or 2D/3D registration in an option.

In one embodiment, 3D imaging is used to create 3D anatomical information of the spine. A 3D model is calculated based on the 3D anatomical dataset, identifying the relevant parts such as the spinal cord, vertebral bodies, cortical bone areas of the vertebral bodies. Optical tracking is used to track the tip of a surgical instrument. This instrument is optionally connected to the electro monitoring system and the electronic measurement location with respect to the tip position is known. The relationship of the relevant anatomical model with its electro-physiological properties with respect to the tracked 3D measurement locations of the neuro-monitoring system is used to improve the neuro-physiological feedback, e.g. reducing false positives and earlier and more robust feedback of true positives.

In another embodiment, 3D imaging is not used; instead multiple 2D fluoro images are used and a 3D anatomical model is matched based on the multitude of 2D images. The 3D anatomical model may use pre-operative CT data or may not use pre-operative 3D data. The position of the surgical instrument is based on a plurality of 2D X-ray images.

The relationship of the relevant anatomical model with its electro-physiological properties with respect to the tracked 3D measurement locations of the neuro-monitoring system is used to improve the neuro-physiological feedback, e.g. reducing false positives and earlier and more robust feedback of true positives.

When neuromonitoring information changes during the insertion of a k-wire or a pedicle screw, it will be automatically known at which depth in the vertebra the problem occurred, and corrective action can be taken.

The resulting neuromonitoring information can be integrated together with procedure information (spatial placement of the screws in the image data) in a report summarizing the procedure and documenting the quality of the procedure.

In an example, an inverse of augmenting the images with monitoring data is provided, where the monitoring signals are augmented with images. For example, if the changes in the signal happen slowly over time or are too small to be detected automatically, the physician may at some point (possibly in the end or after the procedure) notice that something is wrong. The user, i.e. the physician may then scroll through the signals and get corresponding image data for every time point where the physician finds the signal pattern interesting. The processing unit may automatically choose the images that shows most relevant information for that time point.

As an example for a selected time point 1, the camera image of a camera 1 is shown since it documents the movement of a monitoring patch. It does not show an Xray image, because no changes were visible in the X-ray stream at that time point. As an example for a selected time point 2, the camera image of a camera 3 is shown depicting a k-wire 3 being moved. The processing unit does not include the X-ray data since no X-ray was recorded at that time point or since it was recording a different anatomical location than the k-wire 3 which is determined of interest. As an example for a selected time point 3, X-ray images of moving a screw are shown by the processing unit.

In case that, next to the C-arm imaging system, in addition an optical tracking system is used during the procedure, the procedure step, device insertion depth etc. can also be derived from the optical tracking method instead of the X-ray imaging information. When setting up the neurophysiological monitoring system with electrodes outside the main surgical field, their position needs to be manually registered with respect to body part or distance to the point of intervention. In an option, this registration can be automized with a camera-based tracking system, and errors in electrode positioning can be corrected automatically.

When performing e.g. pedicle screw testing by triggered neuro monitoring, such as electromyography, it is important to automatically relate the measurement to the pedicle screw tested. This can be automated using a camera-based tracking system or optical tracking system. Screws which are not tested will be identified automatically.

From a database of procedures combining volumetric and 2D image data of the patient, temporal information on device motion and insertion, e.g. k-wire, pedicle screw, and neuromonitoring information, a network may be trained which will give warnings during the procedure when the risk during the procedure is high or a deterioration of the patient status is to be expected based on the device movement taking place. In case that outcome data of the procedure are available, it will be possible in addition to make intraprocedural suggestions how to optimize the treatment or to interpret if the neuromonitoring signal is an artefact.

Any signal changes that are caused by electrode displacement, patient motion, or objects interfering with the signal acquisition can be identified and the root cause can be shown to the physician. This avoids unnecessary reverse actions on the k-wire or pedicle screw insertion following unrelated signal changes.

Next to an anatomical and device shape model, the visualization of the procedure can be enhancement by a model of the nervous system in the vicinity of the intervention space.

One field of use is minimal invasive spine surgery. Another field of use is ENT surgery (incl. thyroid), vascular surgery (AAA), or neurosurgery (for AAA the anatomical model would be the surface model of the aorta while the device model would be a stent graft being inserted). Whenever one of these procedures is carried out in a hybrid operation room with optical tracking capability, corresponding propositions as described above can be derived.

Examples in which neuromonitoring is applied are minimally invasive procedures such as spinal surgery, treatments of abdominal aortic aneurysms, ENT procedures or selected brain surgeries. An example for such measuring are baseline potential measurements distal to the point of intervention that are used to monitor the spinal cord.

These may include e.g. somatosensory potential measurements, transcranial motor evoked potential measurements, pedicle screw testing by triggered electromyography, or variants thereof. In case there is a change in the baseline signal (for pedicle screw testing - a signal), corrective measures can be taken.

The enhanced image data that is provided helps the user, e.g. during surgery, to aggregate different types of intraoperative neurophysiological signals along their temporal and spatial acquisition axis.

The enhanced image data reduces the complexity of minimal invasive surgical procedures by integration of neurophysiological measurement along the spatial and temporal (procedure step) domain. This also improves procedure safety and efficiency.

In an example, the neurosignal is spatially allocated, i.e. spatially assigned. In an option, the neurosignal is assigned to an additional or supplemental parameter, such as procedural related.

In an option, the neuromonitoring signals are measured at the screw tips.

Fig. 4 shows an example of a display showing neuromonitoring navigation with a visual indicator of the neuromonitoring signals. Fig. 4 shows an example demonstrating an integration of intraprocedural neurophysiological monitoring data on top of an anatomical 3D model and device shape 3D models registered into intraprocedural imaging data. A visually coded indicator, such as a color coded indicator, is provided indicating changes in the neurophysiological data during treatment of respective vertebrae. It is noted that the drawings in black and white reflect the colors by slight greyscale variations only. As an example a first color, such as green, represents no changes, whereas a second color, such as red, represents changes.

Fig. 4 shows a CT based 3D image 200 of an anatomical structure comprising a number of vertebrae 202. Further, 3D models of pedicle screws 204 are indicated inserted in the vertebrae 202. A circular shaped visual indicator 206 is shown within the anatomy forming an augmented image for generating neuromonitoring navigation. A first visual indicator 206a is provided in a first visual coding, such as the color red. A second visual indicator 206b is provided in a second visual coding, such as the color green. A third visual indicator 206c is shown in the second visual coding, i.e. green, whereas a fourth visual indicator 206d is shown in the first visual coding, i.e. red.

Fig. 5 also shows an example of a display showing neuromonitoring navigation with a visual indicator of the neuromonitoring signals. Fig. 5 shows a color coded indicator for changes in the neurophysiological data with respect to a pedicle screw insertion depth. A first color, such as a green, reflects no changes, whereas a second color, such as a green, reflects a change. Fig. 5 shows a tomographic slice 220 of an anatomical structure showing a vertebrae 222. Further, a projected 3D model of a pedicel screw 224 is indicated such that the screw is inserted in the vertebrae 222. A stripe-shaped visual indicator 226 is shown within the anatomy forming an augmented image for generating neuromonitoring navigation. A first part 226a of the visual indicator 226 is provided in the second visual coding, such as the color green. A second part 226b of the visual indicator 226 is provided in the first visual coding, such as the color red. A third part 226c of the visual indicator 226 is provided in the second visual coding, such as the color green.

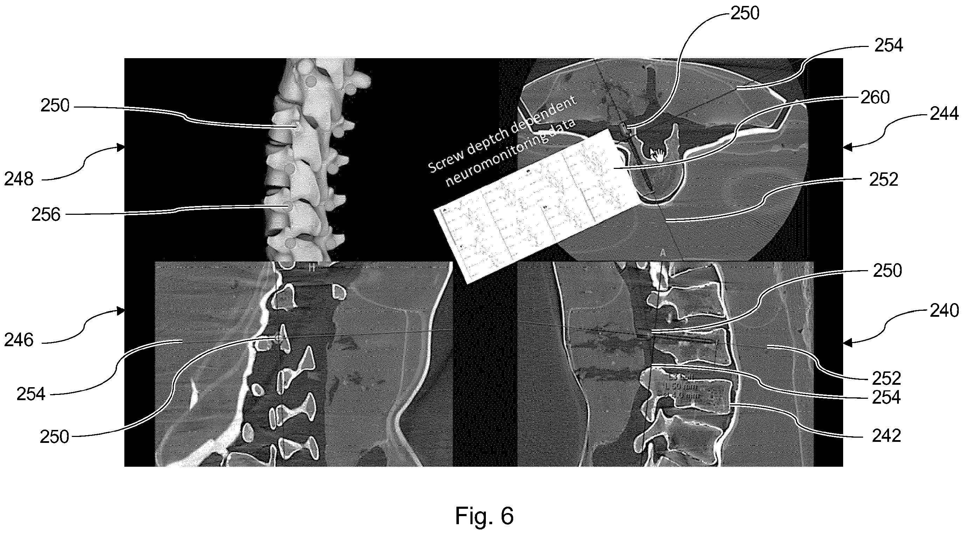

Fig. 6 shows a further example of a display showing neuromonitoring navigation with a visual indicator of the neuromonitoring signals. Fig. 6 shows an example demonstrating the display of screw depth dependent on neuromonitoring data during minimal invasive optically guided screw placement. Fig. 6 shows a first X-ray image 240 of an anatomical structure comprising a plurality of vertebrae 242 in a sagittal or longitudinal view, whereas a second X-ray image 244 shows the anatomical structure in a horizontal, or axial of transverse plane. A third X-ray image 246 shows the anatomical structure in a coronal or frontal plane viewed from the back side. view. A fourth image 248 shows a CTbased rendering type of image of the anatomical structure. A screw 250 is inserted and an operating axis is indicated with a fist line 252. A second line 254 indicates a transverse axis. The screw 250 is highlighted in the fourth image 248. Further screws 256 are also indicated in the fourth image 248. A graph 260 is provided showing screw depth dependent neuromonitoring data in the second image 244.

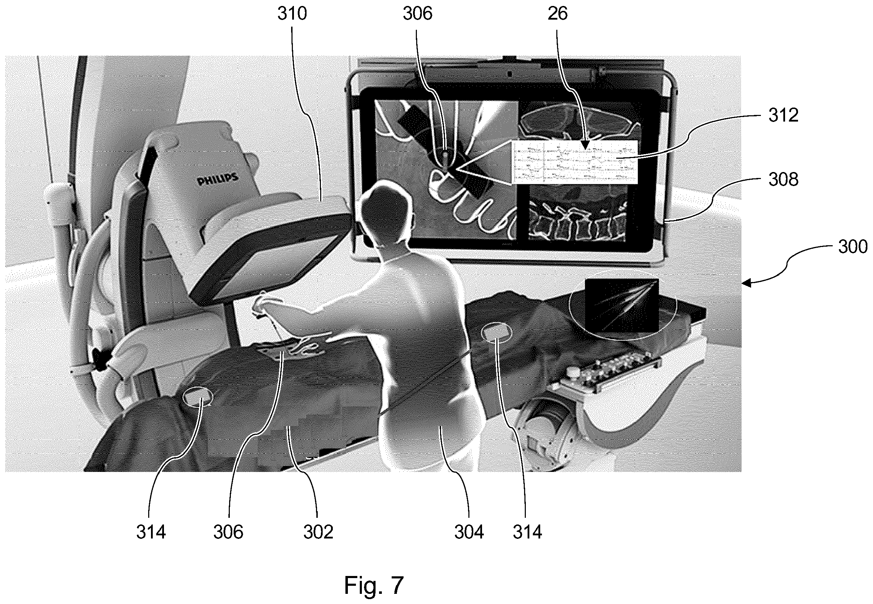

Fig. 7 shows an example illustration demonstrating the positioning of trackable patches and electrodes in the hybrid operating room system leading to screw / k-wire specific display of neuromonitoring signals. Fig. 7 shows a further example of a display showing neuromonitoring navigation with a visual indicator of the neuromonitoring signals. Fig. 7 shows a scenery 300 in an operating room with a subject 302 (under drapes) arranged on a support. A surgeon 304, or any other user, is handling a device 306 like inserting a screw. A monitor arrangement 308 is provided showing different views of a region of interest in which the device is inserted. Image data is provided, among others, from an X-ray imaging system 310. A visual indicator is provided within the anatomy in the augmented image generating the neuromonitoring navigation 26. For example, graphs 312 are provided. Further, patches 314 are indicated which provide the neuromonitoring, but which are also monitored e.g. in their position to make sure that a change in the neuro signal is not caused by a moving of the patches.

In an example, a computer program is provided comprising instructions which, when the program is executed by a computer, cause the computer to carry out the method of the examples above.

In an example, a computer program or program element for controlling an apparatus according to one of the examples above is provided, which program or program element, when being executed by a processing unit, is adapted to perform the method steps of one of the method examples above.

In an example, a computer readable medium is provided having stored the computer program of the previous example.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for neuromonitoring navigation in interventional procedures, the device comprising:
- a data input (12);
- a data processor (14),
wherein the data input is configured:
- to receive a sequence of neuromonitoring signals (18) related to an interventional medical procedure in a region of interest of a subject, the procedure involving at least one interventional device;
- to receive image data representing an anatomy (20) of the region of interest of the subject during the interventional medical procedure; and
- to provide a 3D model (22), the 3D model provided as at least one of the group comprising a 3D anatomical model and a 3D device shape model;
wherein the data processor is configured:
- to register the 3D model to the image data forming an augmented image of the subject;
- to assign the signals of the sequence of neuromonitoring signals to one or more procedural steps of the interventional medical procedure, wherein each procedural step is associated with at least a part of the anatomy;
- to compute a visual indicator of the assigned neuromonitoring signals, and to integrate the visual indicator with the associated part of the anatomy in the augmented image thereby generating neuromonitoring navigation information (24).

2. Device according to claim 1, wherein the data processor is configured to plot the neuromonitoring signals along a procedural axis; and
wherein the visual indicator reflects a correlation of the temporal and the spatial changes.

3. Device according to claim 1 or 2, wherein, for the assigning, the data input is configured to provide procedural data (32) that comprises information about procedural steps of the interventional medical procedure.

4. Device according to claim 1, 2 or 3, wherein the data processor is configured to detect at least one operation axis of the interventional device based on current images; and
wherein the data processor is configured to compute the visual indicator as representing the neuromonitoring signals over a period of operation along the operation axis.

5. Device according to one of the preceding claims, wherein, for the assigning, the data input is configured: for the image data, to provide image data as intraprocedural imaging acquired during the procedure; and
wherein the data processor is configured:
- to identify procedure steps and/or device movements in a field of view based on the intraprocedural imaging; and
- to relate changes in neurophysiological measurements to the procedure steps and/or device movements.

6. Device according to one of the preceding claims, wherein the procedure is a minimally invasive placement of a plurality of pedicle screws; and
wherein the data processor is configured: to identify at least two separate steps of inserting at least one pedicle screw; and to assign separate neuromonitoring signals to each of the at least two separate steps.

7. Device according to one of the preceding claims, wherein the data processor is configured to register at least one model of a vertebra to the imaging information used to guide a device insertion during the procedure.

8. Device according to one of the preceding claims, wherein the data processor is configured to register at least one model of a pedicle screw to the imaging information used to guide a device insertion during the procedure.

9. Device according to one of the preceding claims, wherein the data processor is configured to provide spatial tracking for at least one source by the neuromonitoring signals.

10. Device according to one of the preceding claims, wherein the data processor is configured to detect changes in the neuromonitoring signal exceeding a predetermined threshold and to adapt the visual indicator according to the detected changes; and
wherein the output interface is configured to output an adapted neuromonitoring navigation.

11. A system (50) for neuromonitoring navigation in interventional procedures, comprising:
- a device (10) for neuromonitoring navigation in interventional procedures according to one of the preceding claims;
- an imaging device (52);
- a neuromonitoring device (54); and
- a display device (56);
wherein the imaging device is configured to provide the image data representing the anatomy of the region of interest of a subject (58) during the interventional medical procedure;
wherein the neuromonitoring device is configured to provide the sequence of neuromonitoring signals related to an interventional medical procedure in the region of interest of the subject, the procedure involving at least one interventional device (60); and
wherein the display device is configured to present the neuromonitoring navigation.

12. System according to claim 11, wherein initial imaging is performed prior to the procedure to identify vertebral levels, the initial imaging comprising at least one of the group of 2D, multiple 2D or 3D cone beam X-ray imaging, MR imaging and ultrasound imaging.

13. System according to claim 11 or 12, wherein, to provide the image data, the imaging device is:
i) an X-ray imaging device; and/or
ii) an optical camera device.

14. A method (100) for neuromonitoring navigation in interventional procedures, comprising the following steps:
- receiving (102) a sequence of neuromonitoring signals related to an interventional medical procedure in a region of interest of a subject, the procedure involving at least one interventional device;
- receiving (104) image data representing an anatomy of the region of interest of the subject during the interventional medical procedure;
- registering (106) a 3D model to the image data forming an augmented image of the subject, the 3D model provided as at least one of the group comprising a 3D anatomical model and a 3D device shape model;
- assigning (108) the signals of the sequence of neuromonitoring signals to one or more procedural steps of the interventional medical procedure;
- associating (110) the procedural steps to corresponding parts of the anatomy of the augmented image;
- computing (112) a visual indicator of the assigned neuromonitoring signals and integrating the visual indicator with the associated anatomy in the augmented image thereby generating neuromonitoring navigation information; and
- outputting (114) the neuromonitoring navigation.

15. Computer program comprising instructions which, when the program is executed by a computer, cause the computer to carry out the method of claim 14.
